# EUROPEAN PATENT SPECIFICATION

(11) **EP 3 340 992 B1**
(45) Date of publication and mention of the grant of the patent: **28.12.2022**
(21) Application number: 16843173.2
(22) Date of filing: 05.09.2016
(51) Int. Cl.: A61K 45/06, A61K 31/56, A61P 3/10, A61P 9/10, A23K 20/105, A23K 50/10, A23K 50/40, A23L 33/10

(54) **METHOD FOR TREATMENT OF MONOCYTE DYSFUNCTION AND CHRONIC INFLAMMATORY MICRO-AND MACRO-VASCULAR DISEASES**
VERFAHREN ZUR BEHANDLUNG VON MONOZYTENDYSFUNKTION UND CHRONISCHEN ENTZÜNDLICHEN MIKRO- UND MAKROVASKULÄREN ERKRANKUNGEN
MÉTHODE POUR LE TRAITEMENT D'UN DYSFONCTIONNMENT DES MONOCYTES ET DES MALADIES MICROVASCULAIRES ET MACROVASCULAIRES INFLAMAMTOIRES CHRONIQUES

(30) Priority: 04.09.2015 US 201562214465 P
(43) Date of publication of application: 04.07.2018
(73) Proprietor: The Board of Regents of the University of Texas System, Austin, TX 78701 (US)
(72) Inventor: Asmis, Reto, San Antonio, TX 78229 (US)
(74) Representative: FRKelly
(86) International application number: PCT/US2016/050332
(87) International publication number: WO 2017/041077

(56) References cited:
- KR-B1- 101 278 273
- US-A1- 2012 077 761
- S.L. ULLEVIG, ET AL.: "Ursolic acid protects diabetic mice against monocyte dysfunction and accelerated atherosclerosis", ATHEROSCLEROSIS, vol. 219, no. 2, 17 June 2011 (2011-06-17) , pages 409-416, XP028121256, Elsevier Ireland, Shannon, IE ISSN: 0021-9150, DOI: 10.1016/j.atherosclerosis.2011.06.013
- QIN ZHANG, ET AL.: "Ursane Triterpenoids Inhibit Atherosclerosis and Xanthoma in LDL Receptor Knockout Mice", CARDIOVASCULAR DRUGS AND THERAPY, vol. 20, no. 5, 29 November 2006 (2006-11-29), pages 349-357, XP019447714, Kluwer Acadamic Publishers, Boston, US ISSN: 1573-7241, DOI: 10.1007/S10557-006-0509-4
- ULLEVIG ET AL.: 'Ursolic Acid Protects Diabetic Mice Against Monocyte Dysfunction and Accelerated Atherosclerosis' ATHEROSCLEROSIS. vol. 219, no. 2, 2011, pages 409 - 416, XP028121256
- TAPONDJOU ET AL.: 'In vivo anti-nociceptive and anti-inflammatory effect of the two triterpenes, ursolic acid and 23-hydroxyursolic acid, from Cussonia bancoensis' ARCH PHARM RES. vol. 26, no. 2, 01 February 2003, pages 143 - 146, XP053006969 DOI: 10.1007/BF02976660
- ZHANG ET AL.: 'Ursane triterpenoids inhibit atherosclerosis and xanthoma in LDL receptor knockout mice' CARDIOVASC DRUGS THER vol. 20, no. 5, 2006, pages 349 - 357, XP019447714
- SHIN ET AL.: 'In vitro antiinflammatory activity of 23-hydroxyursolic acid isolated from Cussonia bancoensis in murine macrophage RAW 264.7 cells.' PLANTA MED. vol. 70, no. 9, 2004, pages 803 - 807, XP055517595 DOI: 10.1055/S-2004-827226

## Description

This application claims priority to U.S. Provisional Application serial number 62/214,465 filed September 4, 2015.

This invention was made with government support under Grant No. AT006885 awarded by National Institutes of Health. The government has certain rights in the invention.

### 1. Field of the Invention

The present invention relates generally to the field of medicine and physiology. More particularly, it concerns compositions comprising 23 hydroxy ursolic acid or a pharmaceutically acceptable salt thereof for use in methods for treating vascular diseases and conditions.

### 2. Background

The earliest events in atherosclerosis involve the recruitment of monocytes initiated by the expression of adhesion molecules by endothelial cells lining the vascular wall and the release of chemoattractant chemokines by the vasculature. Early lesions are characterized by the accumulation and prolonged persistence of monocyte-derived macrophages in the subendothelial space and their transformation into lipid-laden foam cells (Libby, Nature, 420:868-74 (2002)). Studies utilizing monocyte chemoattractant protein-1 (MCP-1) or CCR2-deficient mice demonstrated that MCP-1 and its receptor, CCR2, are intricately involved in the initiation and development of atherosclerosis (Boring et al. Nature, 394:894-97, (1998)). Although other chemokine/chemokine receptor pairs have recently been implicated in the development and progression of atherosclerosis, including fractalkine (CXCL13)/CXCR13) and RANTES (CCL5)/CCR5), MCP-1/CCR2 are central to all stages of atherogenesis (Gautier et al. Arterioscler Thromb Vasc Biol., 29:1412-18 (2009)).

Diabetes is a metabolic disease characterized by chronic hyperglycemia and insulin resistance, which lead to macro- and microvascular complications, including atherosclerosis, peripheral vascular disease, nephropathy, retinopathy, and neuropathy (Lamb and Goldstein Int J Clin Pract., 62:1087-95(2008)). Accelerated atherosclerosis is a major complication of diabetes, with diabetics having a 2-4 fold higher rate of mortality from heart-related complications than non-diabetics (McEwen et al. Diabetes Care, 29:247-53 (2006)), but the mechanisms that lead to accelerated atherosclerotic lesion formation in diabetics are not fully understood. Many metabolic diseases, including diabetes, have been linked to increased oxidative stress, the over-activation of the immune system (Lamb and Goldstein Int J Clin Pract., 62:1087-95(2008); Brownlee Nature, 414:813-20, (2001)) and the dysregulation of monocyte and macrophage functions (Tesch Clin Exp Pharmacol Physiol., 34:1016-19 (2007)). For example, monocytes isolated from diabetic patients show alterations in cell metabolism (Rosen et al. Diabetes Metab. Res. Rev., 17:189-212 (2001)), phagocytosis (Padmos et al. Diabetes, 57:2768-73 (2008)) and cytokine production and release (Noritake et al. Clin. exp. Immunol., 88:269-74 (1992); Ohno et al. J Clin Endocrinol Metab., 77:1072-77 (1993)), and similar changes in cytokine release and morphology have been reported in macrophages isolated from diabetic mice. In a mouse model of diabetic complications, it was found that the diabetic condition not only increased the severity of atherosclerosis but atherosclerotic lesion size tightly correlated with increased chemotactic activity of blood monocytes *in vivo* and increased macrophage recruitment into sites of vascular and renal lesions (Qiao et al. Arterioscler Thromb Vase Biol., 29:1779-86 (2009)). Accelerated monocyte transmigration and macrophage recruitment in turn was associated with increased intracellular thiol oxidative stress in these cells.

A number of anti-inflammatory phytonutrients have shown promise in the prevention and treatment of diabetic complications and thus may represent an affordable alternative to more traditional anti-diabetic drugs and therapies (Omar et al. Curr Pharm Des., 16:3776-3807 (2010); Hirai et al. Mediators Inflamm., 2010:367838 (2010)). There remains a need for additional compositions for use in methods for treating vascular and diabetic complications, particularly those complications associated with dysregulation of monocyte activity.

### SUMMARY

Many metabolic diseases, including diabetes, have been linked to increased oxidative stress, the over-activation of the immune system, and the dysregulation of monocyte and macrophage functions. For example, monocytes isolated from diabetic patients show alterations in cell metabolism, phagocytosis, and cytokine production and release. Similar changes in cytokine release and morphology have been reported in macrophages isolated from diabetic mice. In a mouse model of diabetic complications, it was found that the diabetic condition not only increased the severity of atherosclerosis, but atherosclerotic lesion size tightly correlated with increased chemotactic activity of blood monocytes *in vivo* and increased macrophage recruitment into sites of vascular and renal lesions. Accelerated monocyte transmigration and macrophage recruitment in turn was associated with increased intracellular thiol oxidative stress in these cells. As described herein, the inventor has examined the effect of 23 hydroxy ursolic acid (23-OH UA; 3β, 23-dihydroxyurs-12-en-28oic acid) and its effectiveness as an anti-diabetic and/or anti-atherogenic and/or anti-obesogenic.

The present disclosure concerns pharmaceutical compositions for use in methods useful for the treatment of conditions related to, caused by, or a result of monocyte hypersensitivity or hyperactivity, such as diabetes and atherosclerosis, as well as other conditions and vascular conditions. The pharmaceutical compositions for use of the present invention utilize 23 hydroxy ursolic acid (formula I) in a method of reducing the risk of vascular disease by attenuating monocyte hypersensitivity in a subject.

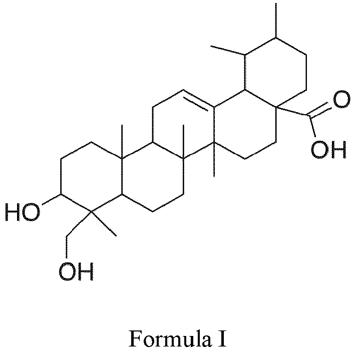

The invention is directed to pharmaceutical compositions for use in methods for treating conditions related to, caused by, or is a result of monocyte hypersensitivity in a subject by administering to the subject a pharmaceutical composition comprising 23 hydroxy ursolic acid or a pharmaceutically acceptable salt thereof. Monocyte hypersensitivity may be associated with diabetes, atherosclerosis, obesity, and/or other vascular conditions. As claimed herein, the compositions are for use in a method of reducing the risk of vascular disease by attenuating monocyte hypersensitivity in a subject. Pharmaceutical compositions may be administered 1, 2, 3, 4, 5, 6, 7, 8, 9, 10, 11, 12, 13, 14, 15, 16, 17, 18, 19, 20 or more times, and they may be administered every 1, 2, 3, 4, 5, 6, 7, 8, 9, 10, 11, 12, 13, 14, 15, 16, 17, 18, 19, 20, 21, 22, 23, 24 hours, or 1, 2, 3, 4, 5, 6, 7 days, or 1, 2, 3, 4, 5 weeks, or 1, 2, 3, 4, 5, 6, 7, 8, 9, 10, 11, 12 months or longer. 23-OH UA and UA inhibit monocyte "priming" by metabolic stress, that is the hypersensitization of monocytes to chemokine-induced activation, which can result in accelerated chemotaxis/migration. These compounds do not block or affect normal or baseline responses to chemokines, leaving normal, non-pathogenic chemotaxis intact, whereas inhibitors of normal chemotaxis would have an "immunosuppressive" effect. The UA compounds only block the pathogenic component of the signal, the hypersensitivity induced by metabolic disorders, but leave normal migration untouched.

Some embodiments are directed to animal feed, food compositions, pharmaceutical compositions or dietary supplements that comprise 23 hydroxy ursolic acid. In certain aspects the food composition is an animal feed or food fit for human consumption.

Pharmaceutical compositions for use in methods described herein are disclosed, directed to treating complications of diabetes (type I, type II or gestational diabetes). In some examples, treatment encompasses alleviation of symptoms and/or delay in disease progression.

In certain aspects the subject is a human subject. The subject may be diagnosed or at risk of developing diabetes, e.g., a pre-diabetic. The subject may be obese. The subject may be diagnosed with or at risk of developing atherosclerosis, e.g., the subject has high cholesterol and/or triglycerides and/or high levels of free fatty acids and/or hyperglycemia and/or monocytosis. The subject at risk has monocyte hypersensitivity.

The pharmaceutical composition for use in a method of the present invention may be combined with additional treatment or treatments. In some embodiments, 23 hydroxy ursolic acid is administered in combination with another therapeutic agent, for example, an anti-atherogenic agent. Also disclosed is the administration of 23 hydroxy ursolic acid in combination with an anti-inflammatory, an anti-diabetic, an anti-obesogenic, or anti-atherosclerotic agent.

In certain embodiments a subject can be at risk of developing atherosclerosis. A subject may be identified as at risk by using various risk assessment components that include, but are not limited to diagnosis with diabetes or pre-diabetes, genetic predisposition, biomarker analysis, and imaging.

Certain embodiments are directed to a dietary supplement comprising 10, 20, 30, 40, 50 to 60, 70, 80, 90% by weight 23 hydroxy ursolic acid, including all values and ranges there between. Other aspects are directed to methods of supplementing a diet of an animal or human comprising administering the dietary supplement comprising or containing 23-OH UA. A further aspect is directed to an animal food composition comprising 0.1,1, 2, 3, 4, 5, to 6, 7, 8, 9, 10% of 23 hydroxy ursolic acid or more; disclosed is an animal food composition comprising up to 20, 30, 40, 50 %. The composition can be a pet or livestock food.

Also disclosed are pharmaceutical compositions for use in methods of reducing the risk of vascular disease in a subject, a diabetic subject, or a pre-diabetic subject comprising administering to the subject, diabetic subject, or pre-diabetic subject an effective amount of a pharmaceutical composition comprising 23 hydroxy ursolic acid or a pharmaceutically acceptable salt thereof. In certain aspects the vascular disease is atherosclerosis, peripheral artery disease, coronary heart disease (CHD). Other vascular diseases associated with monocyte dysregulation are disclosed.

As used herein, the term "IC₅₀"refers to an inhibitory dose that results in 50% of the maximum response obtained.

The term half maximal effective concentration (EC₅₀) refers to the concentration of a drug that presents a response halfway between the baseline and maximum after some specified exposure time.

The terms "amelioration", "inhibiting", "reducing," or "prevention," or any variation of these terms, when used in the claims and/or the specification includes any measurable decrease or complete inhibition to achieve a desired result.

As used herein the specification, "a" or "an" may mean one or more. As used herein in the claim(s), when used in conjunction with the word "comprising", the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the detailed description and the specific examples, while indicating preferred embodiments of the invention, are given by way of illustration only, since various changes and modifications within the scope of the invention will become apparent to those skilled in the art from this detailed description.

### DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

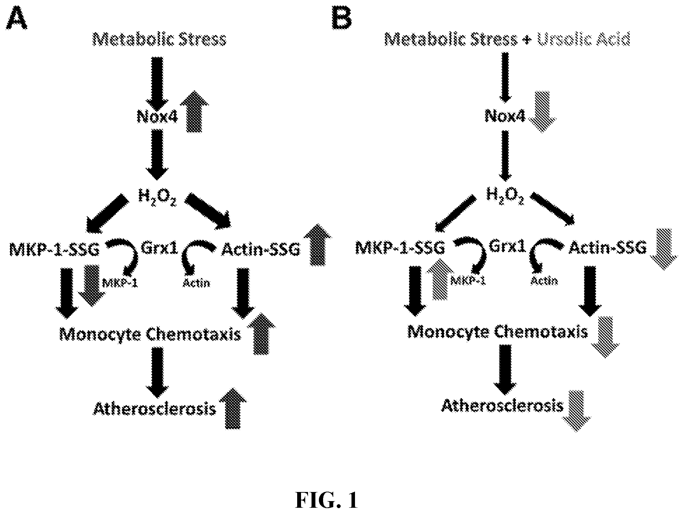

**FIG. 1** illustrates mechanisms involved monocyte priming by metabolic stress resulting in an increase in monocyte chemotaxis (A) and the effect of UA and 23-OH UA on attenuating the metabolic stress induced monocyte chemotaxis (B).

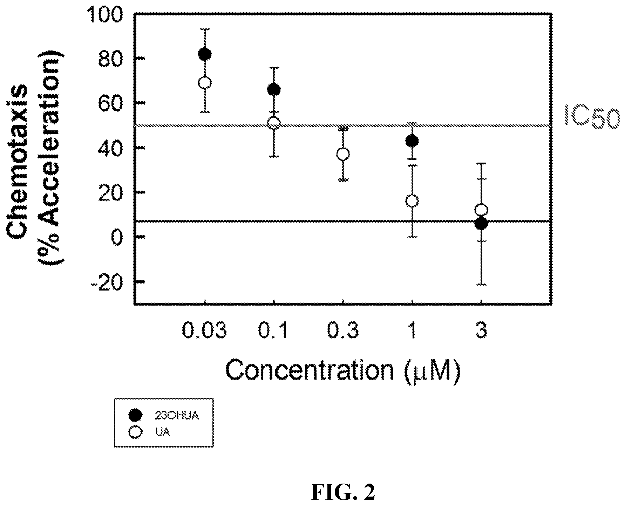

**FIG. 2** shows the effectiveness of UA and 23OH UA in reducing monocyte chemotaxis. Chemotaxis assays are performed using 48-well, modified Boyden chambers (NeuroProbe, Gaithersburg, MD) as described previously (1). Briefly, monocytes are primed with glucose and LDL in the presence of UA, 23-OHUA or vehicle (0.1% DMSO). Cells are loaded into upper wells of the chemotaxis chamber. Lower wells contain cell media with 1 nM MCP-1 (R&D Systems, Minneapolic, MN) or vehicle. A 5 µm polyvinyl pyrrolidone-free polycarbonate filter membrane is layered between the upper and lower chambers, and the chamber is incubated for 1.5 h for THP-1 monocytes at 37 °C and 5% CO2. The membrane is washed and cells removed from the upper side of the filter and fixed with methanol. Transmigrated are stained with 1 µM propidium iodide. Fluorescence intensity, which correlates with cell number is quantified with KODAK Image Station 4000MM (Carestream, Rochester, NY).

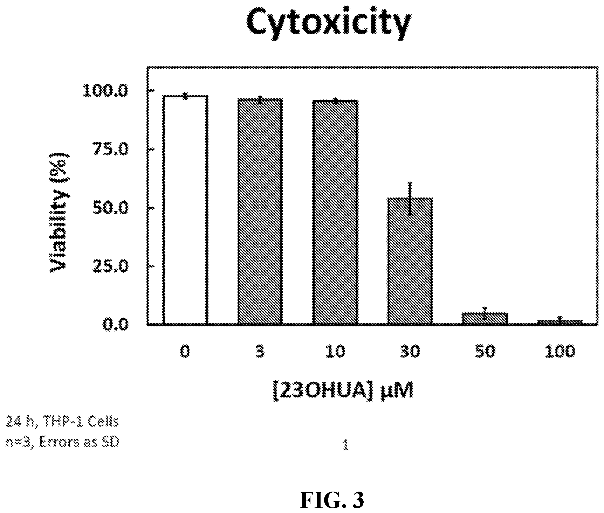

**FIG. 3** shows the cytoxicity of 23-OH UA in THP-1 cells. Cytotoxicity was determined by Trypan blue staining.

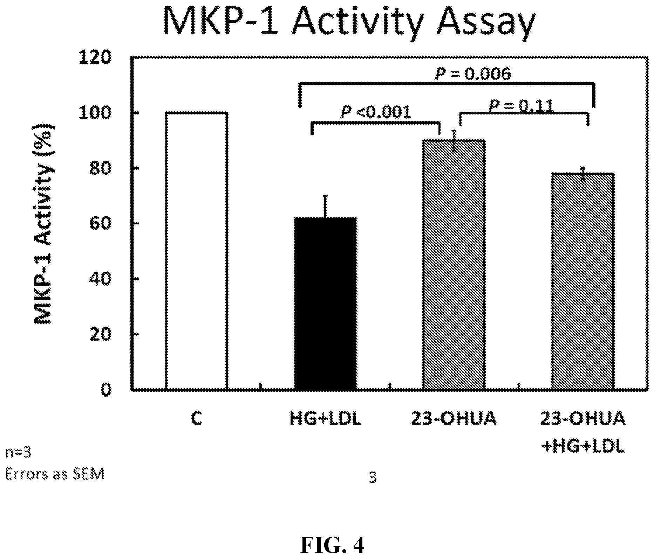

**FIG. 4** shows the effect of 23-OH UA on MKP-1 activity.

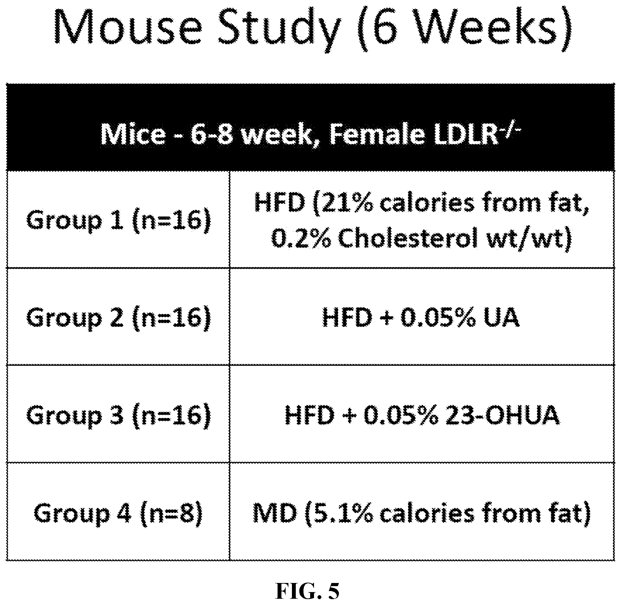

**FIG. 5** summarizes a mouse study design for further characterization of 23-OH UA. Female LDL-R^{-/-} recipient mice (B6.129S7-Ldlr^{tm1her}/J, stock no. 002207) were obtained from Jackson Labs (Bar Harbor, ME). All mice were maintained in colony cages on a 12-h light/12-h dark cycle. Mice were randomly assigned to one of four groups, (8 mice per group at 6 and 20 weeks) high fat diet (HFD; 21% milk fat and 0.2% cholesterol, diet no. F5540, Bio-Serv, Frenchtown, NJ), HFD supplemented with 0.05% ursolic acid, HFD supplemented with 0.05% 23-OHUA or maintenance diet (MD; AIN-93G, Bio-Serv). Phytonutrient diets were prepared by Bio-Serv. Mice were maintained on diets for 6 or 20 weeks. Fasted body weights were assessed weekly and fasted glucose was assessed by venous tail bleed biweekly. At 6 and 20 weeks, complete blood counts were run using whole blood. All studies were performed in accordance with the guidelines and regulations of and with the approval of the UTHSCSA Institutional Animal Care and Use Committee.

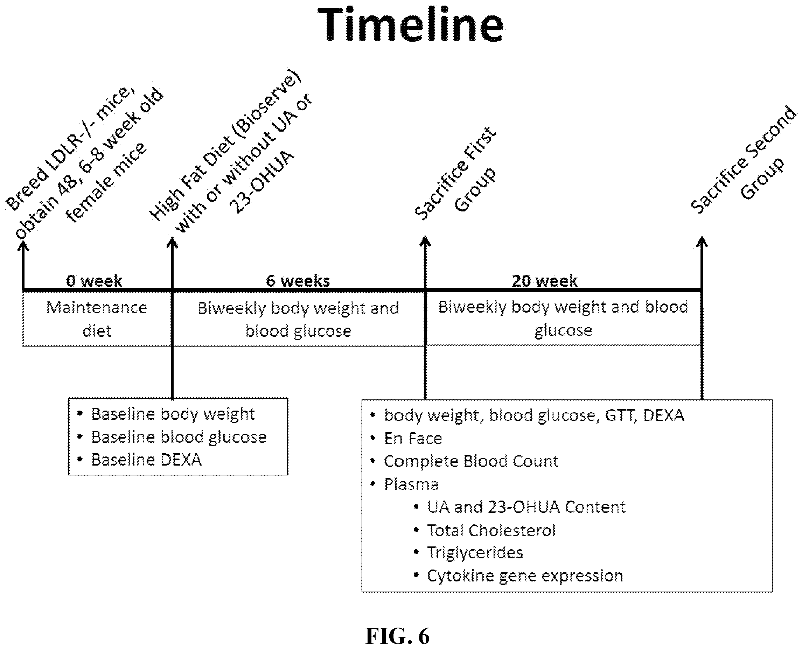

**FIG. 6** summarizes the study protocol for the mouse study designed for further characterization of 23-OH UA. See FIG. 5.

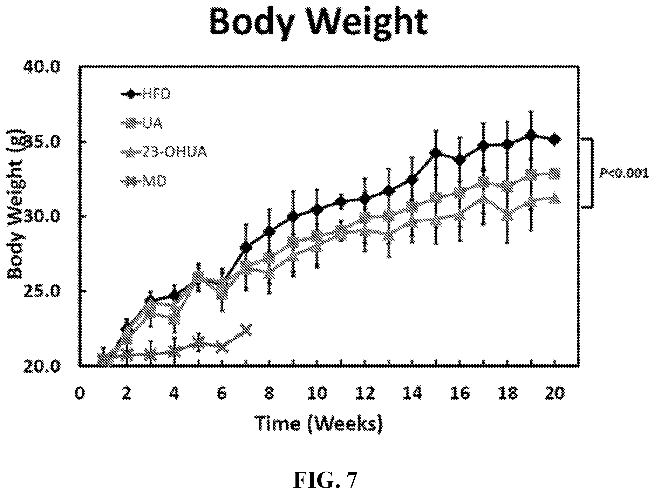

**FIG. 7** shows the comparative effect of 23-OH UA on body weight with high fat diet and UA. See FIG. 5.

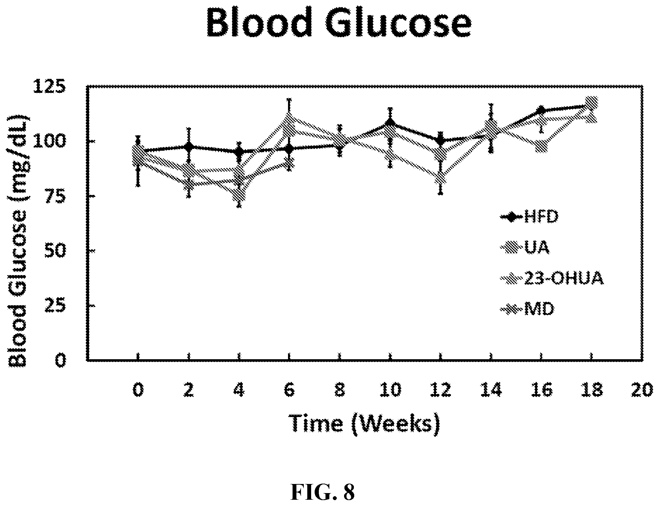

**FIG. 8** shows the comparative effect of 23-OH UA on blood glucose with high fat diet and UA. See FIG. 5.

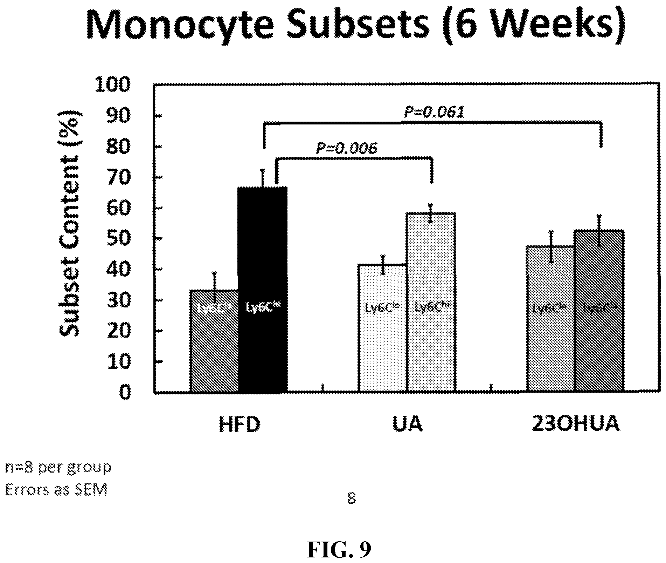

**FIG. 9** shows the monocyte subsets at 6 weeks in HFD, UA, and 23-OH UA groups. For the identification and quantification of monocyte subsets, whole blood was incubated in FACS buffer at 4°C, for 15 min to block F_{C} receptors (CD16/CD32). Red blood cells (RBC) were lysed with 2 ml of BD FACS lysing solution (BD Biosciences) and subsequently labeled with V450-ly-6G (BD Horizon), APC-efluor 780 ly-6C (eBioscience), PE-CD115 (eBioscience), and Alexa Fluor 488-CD11b (BioLegend) antibodies in FACS buffer. Cells were fixed and permeabilized with 2% PFA in PBS for 30 min at 4°C. Analysis was performed using a BD LSR-II.

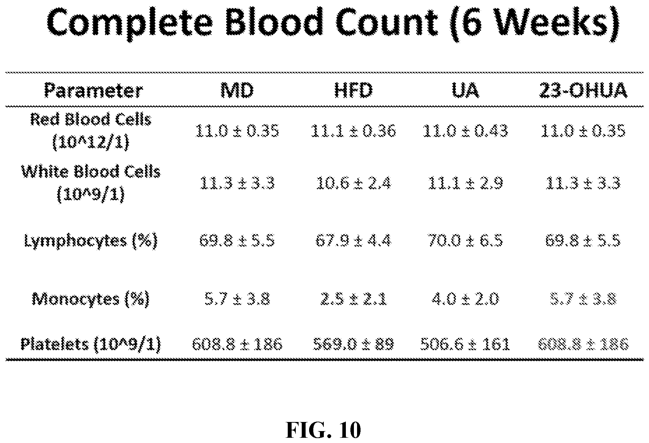

**FIG. 10** shows the blood count results at 6 weeks in HFD, UA, and 23-OH UA groups. For differential blood cell counts, cardiac blood was collected and blood cell counts were obtained on an Abaxis VetScan HM2 Complete Blood Count Analyzer.

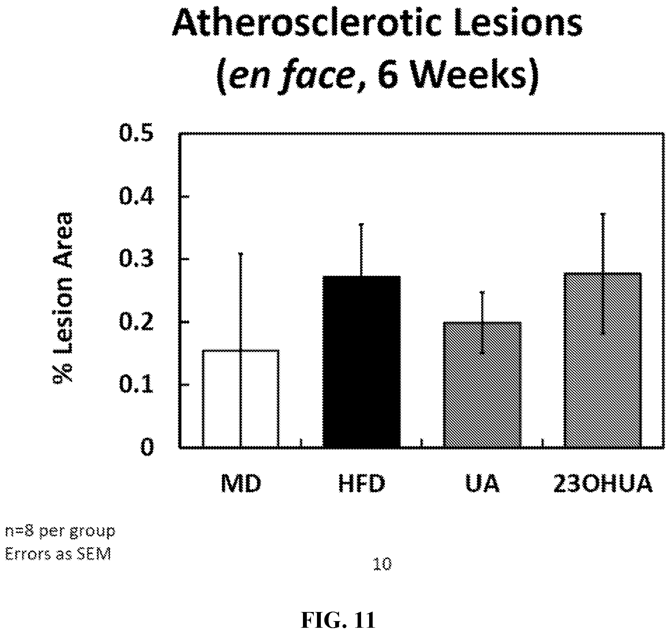

**FIG. 11** shows the effect on atherosclerotic lesions at 6 weeks of HFD, UA, and 23-OH UA. The chest cavity was opened and the heart and aorta were perfused via the left ventricle with 10 ml PBS followed by 10 ml of ice-cold 4% paraformaldehyde (PFA) in PBS. With the heart intact, the entire aorta (extending 5 mm after bifurcation of the iliac artery, including the subclavian artery, right, and left carotid arteries) was dissected free of fat and removed. Hearts were separated from the aorta and embedded in Tissue-Tek^{®} Optimal Cutting Temperature compound (OCT; SAKURA Finetek USA, Inc., Torrance, CA) in a plastic cryosection mold. The aortas (proximal ascending aorta to the bifurcation) were fixed in 4% PFA prior to staining for *en face* analysis. To determine the extent of the atherosclerosis, aortas were stained with Oil Red O (ORO; Sigma-Aldrich, St. Louis, MO) and digitally imaged with a camera connected to a dissecting microscope (Leica). Images were taken to include the entire aorta. Next, the aortas were opened longitudinally and digitally imaged at fixed magnification. Total aortic area and lesion area were calculated using Image Pro Plus (version 6.3; Media Cybernetics, Warrendale, PA) and ImageJ (version 1.47; NIH, Bethesda, Maryland). Lesion area is expressed as a percent of total aortic area ± S.E.

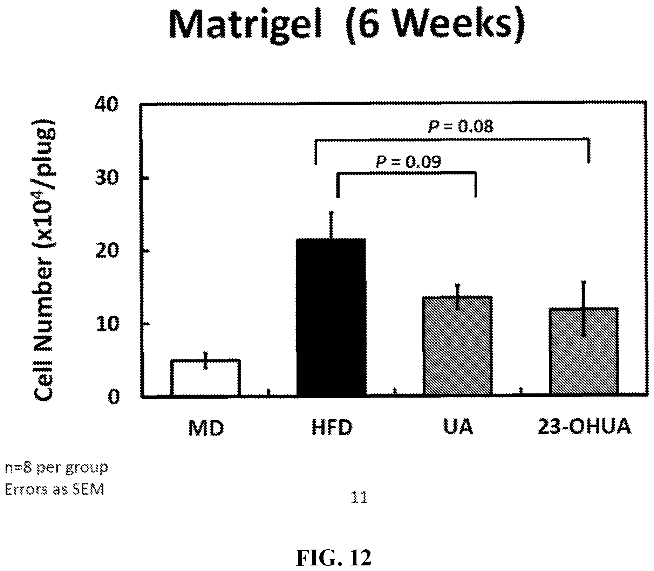

**FIG. 12** shows the effect on cell invasiveness at 6 weeks in HFD, UA, and 23-OH UA. The Matrigel plug assay we developed was used to determine monocyte chemotaxis (or "invasiveness") in response to chemoattractant (MCP-1) *in vivo.* Matrigel (BD Biosciences) supplemented with vehicle (injected in mouse's left flank) or MCP-1 (300 nM, injected in mouse's right flank) is injected subcutaneously. After 3 days, mice were sacrificed and the plugs removed. Plugs are dissolved in collagenase for 2 hours at 37°C. Cells were stained with calcein/AM (Invitrogen, Grand Island, NY) and counted using an automated fluorescent cell counter (Nexcelcom Bios, Lawrence, MA).

### DETAILED DESCRIPTION

The inventor has examined the effect of ursolic acid, an anti-inflammatory triterpenoid with well-documented anti-tumor properties (Ikeda et al. Mol. Nutr. Food Res., 52:26-42 (2008)) and compared its effectiveness in preventing accelerated atherosclerosis to that of resveratrol (RES), a phytonutrient with well-established anti-diabetic (Penumathsa et al. J Cell Mol Med., 12:2350-61 (2008) ; Thirunavukkarasu et al. Free Radic. Biol. Med., 43:720-29 (2007)), anti-atherogenic (Do et al. Biochem Biophys Res Commun., 374:55-59 (2008)), and cancer preventive properties (Shukla and Singh Ann N YAcad Sci., 1215:1-8 (2011)).

RES, a stilbene found in grapes, wine, peanuts, and other plant sources (Baur and Sinclair Nat Rev Drug Discov., 5:493-506 (2006)), has been utilized in many studies focused on the treatment of diseases such as diabetes, atherosclerosis, and cancer (Harikumar and Aggarwal Cell Cycle, 7:1020-35 (2008)). RES was shown to scavenge reactive oxygen species (ROS) and induce antioxidant enzymes in a number of systems (Ungvari et al. Am. J. Physiol. Heart Circ. Physiol., 292:H2417-H2424 (2007)). Many of RES' beneficial properties are modulated through the indirect activation of Sirtuin 1 (SIRT1) (Pacholec et al. J Biol Chem., 285:8340-51 (2010); Pervaiz and Holme Antioxid. Redox Signal., 11:2851-97 (2009)), a histone deacetylase. In addition to its role in aging, SIRT1 mediates RES' ability to inhibit NF-κB, a thiol sensitive transcription factor that controls pro-inflammatory responses (Harikumar and Aggarwal Cell Cycle., 7:1020-35 (2008); Pervaiz and Holme Antioxid. Redox Signal., 11:2851-97 (2009)). This mechanism may account for the reported anti-inflammatory effects of RES in macrophages (Yoshizaki et al. Am J Physiol Endocrinol Metab., 298:E419-28 (2010)).

Ursolic acid (UA) is a pentacyclic triterpenoid found in many herbs and spices like rosemary and thyme, but also in fruits including apples, cranberries, and blueberries. UA has been primarily studied as an anti-cancer and anti-inflammatory compound. More recently, however, dietary supplementation with UA was found to improve glycemic control and lipid profiles, to decrease lipid accumulation in the liver and increase antioxidant enzymes activity in rodent models of metabolic disease (Somova et al. Phytomedicine, 10:115-21 (2003); Jayaprakasam et al. J Agric Food Chem., 54:243-48 (2006)). However, the effect of dietary UA on diabetic complications, particularly accelerated atherosclerosis, has not been investigated. The inventor has shown that UA is a potent inhibitor of diabetic atherosclerosis and provide evidence that UA's anti-atherogenic activity at least to a large extent appears to involve protecting of monocytes from hyper-reactivity to MCP-1 induced by metabolic stress and accelerated cell migration.

Dietary supplementation with ursolic acid attenuates monocyte dysfunction and macrophage recruitment and protects diabetic mice from atherosclerosis. The inventor has elucidated the mechanism through which hypercholesterolemia and hyperglycemia promote monocyte dysfunction. Monocyte dysfunction is induced by metabolic disorders via the pathological induction of Nox4. This pathological induction of Nox4 results in (ROS (H₂O₂) formation and the S-glutathionylation of a large number of proteins involved in cell signaling and key cellular functions. In particular, MKP-1, which is then S-glutathionylated and degraded. The inventor has shown that MKP-1 deficiency in monocytes is sufficient to account for the proinflammatory and proatherogenic phenotype that is induced by metabolic stress, both *in vitro* and *in vivo.* As described herein, ursolic acid and several of its structural analogues prevent the induction of Nox4 (and subsequently pathological S-glutathionylation and inactivation/loss of MKP-1), thus protecting monocytes from metabolic stress induced dysfunction.

Structure-function studies have identified 23 hydroxy ursolic acid (23-OH UA) as a potent, more soluble compound with a similar mechanism of action as ursolic acid. Data indicates that 23-hydroxy ursolic acid also prevents the induction of Nox4 and monocyte dysfunction. Attenuation of monocyte dysfunction can be used to regulate monocyte-driven (chronic) inflammatory diseases associated with metabolic disorders, including diabetic nephropathy, and thus possibly also diabetic retinopathy, aortic aneurysms, and impaired wound healing.

Evidence indicates that 23-OH-UA works via the same mechanism of action as UA, the parent compound (Ullevig et al. Redox Biology. 2, 259-66 (2014); Ullevig et al. Atherosclerosis 219, 409-16 (2011); Kim et al. Arterioscler. Thromb.Vasc. Biol. 34, 1514-21 (2014); Lee et al. PLOS ONE June 18, 8(6):e66964. (2013); Kim et al. Proc. Natl. Acad. Sci. USA, 109, E2803-12 (2012); Ullevig et al. Arterioscler. Thromb. Vasc. Biol. 32, 415-26 (2012)). UA is well-tolerated by rodents and humans and it lowers blood glucose levels in mice, but does not affect lipid levels. Zhan, et al. discloses that ursane triterpenoids inhibit atherosclerosis and xanthoma in LDL receptor knockout mice (Cardiovascular Drugs and Therapy, (20061129), vol. 20, no. 5, pages 349 - 357).

In certain aspects of the invention 23-OH UA can work synergistically with anti-atherogenic drugs, such as statins. 23-OH UA can also work synergistically with anti-diabetic drugs/insulin sensitizers and the like. Animal studies have been conducted with 23-OH UA to characterize its efficacy and bioavailability (and its glucose lowering affect). In certain embodiments 23-OH UA is administered in combination with statins. 23-OH UA may be administered in combination with anti-diabetic drugs. In certain aspects, 23-OH UA is co-formulated with an anti-atherogenic drug. 23-OH UA may be co-formulated with an anti-diabetic drug.

23-OH UA can be highly effective in the prevention of monocyte dysfunction associated with metabolic disorders and chronic inflammatory diseases, particularly atherosclerosis and macro- and microvascular complications associated with diabetes. 23-OH UA is more hydrophilic and thus can have an increased bioavailability as an oral or dietary supplement relative to UA. 23-OH UA or its analogs can be used for combination therapy in diabetes or in people with an increased or high risk for atherosclerosis and CVD

Certain embodiments are directed to pharmaceutical compositions for use in methods for treating or reducing the risk of developing disorders associated with monocyte dysregulation by administering 23-OH UA to a subject at risk of having such disorder. As used herein, "at risk" refers to individuals who have a high probability of developing atherosclerosis. The use comprises the step of administering to an individual with or at risk of monocyte associated disorder, an effective amount of 23-OH UA. The risk assessment of a subject can comprise, but is not limited to assessment of disease diagnosis (diabetes or prediabetes), genetic predisposition, biomarker analysis, and atherosclerosis imaging. Other factors that affect subject's risk of developing CVD or atherosclerosis include sex, age, diabetes, smoking, blood pressure, total cholesterol and LDL cholesterol (Wilson, Circulation, 1998, 97:1837-47).

*Diabetes*/*Prediabetes.* Diabetes mellitus, often just called diabetes, is a condition in which a person's body does not produce enough, or does not properly respond to, insulin. Insulin is a hormone produced in the pancreas that enables cells to absorb glucose to turn it into energy. When insulin production is insufficient or when the body does not properly respond to insulin, glucose accumulates in the blood, which can lead to various complications. While there are several forms of diabetes, three forms are the most recognized: type I diabetes, type II diabetes, and gestational diabetes. Additionally, prediabetes is recognized as preceding diabetes and exists when blood glucose levels that are higher than normal but not yet high enough to be diagnosed as diabetes.

Type I diabetes, which affects about 5-10 percent of Americans diagnosed with diabetes, is a metabolic disorder that is caused by destruction of the insulin-producing beta cells in the pancreas which leads to insulin deficiency and high levels of glucose in plasma. The onset of type I diabetes generally results from an autoimmune etiology; however, idiopathic causes of beta cell destruction can occur for type I. Type 1 diabetes can affect children or adults, but was traditionally termed "juvenile diabetes" because it represents a majority of the diabetes cases in children.

Type II diabetes is similar to other forms of diabetes as it presents with high levels of plasma glucose and is correlated with metabolic abnormalities. Generally, type II diabetes is characterized by insulin resistance, which also may be combined with reduced insulin secretion. The onset of type II diabetes is often gradual, and in the early stages of type II diabetes, the predominant abnormality is reduced insulin sensitivity. As type II diabetes progresses, the ability of the pancreas to secrete insulin is affected. Type II diabetes is the most common type of diabetes.

Gestational diabetes occurs in pregnant women who have not previously been diagnosed with diabetes but who have high glucose levels during pregnancy. Gestational diabetes affects about 4% of all pregnant women and may precede development of type II diabetes.

As the rate of obesity has increased in the United States, the prevalence of diabetes has increased as well. It has been reported that approximately 19 million Americans suffer from type II diabetes. In 1990, only 4.9% of individuals 18 years of age or older in the United States reported having diabetes. In 2001, however, 7.9% reported having diabetes. Patients considered obese saw the greatest prevalence of diabetes, with 14.9% of persons having a 35-39.9 kg/m² BMI and 25.6% of persons having a >40 kg/m² BMI suffering from diabetes.

*Genetic Predisposition.* The genetic predisposition includes information on family history as well as the detection of one or more genetic polymorphisms or mutations associated with increased cardiovascular disease (CVD) risk. Family history is an important and independent CVD risk factor, especially for early onset disease. Many studies have found a two to three-fold increase in CVD given a first-degree relative with CAD (Slack et al. J. Med Genet 1966, 3:239-237; Friedlander et al. Br Heart J 1985, 53:383-387; Thomas et al. Ann Intern Med. 1955; 42:90-127; Lloyd-Jones et al. JAMA 2004; 291:2204-2211).

The family history evaluation is conducted by interview with the subject. The subject can be considered to have a family history of CVD if there is a family history of premature CHD (MI or sudden death before age 55 in father or other male first-degree relative, or before age 65 in mother or other female first-degree relative). In one aspect, genetic predisposition comprises one or more of a family history value, an ApoE4 mutation, an Apo E2 mutation, a LIPC-480 C/T mutation, a LIPC-514 C/T mutation, or a 5-lipoxygenase polymorphism.

Lipoprotein levels are determined by genes that code for proteins that regulate lipoprotein synthesis, interconversions and catabolism. These include the apolipoproteins, the lipoprotein processing proteins and the lipoprotein receptors. There are six major classes of apolipoproteins and several subclasses including: A (apo A-I, apo A-II, apo A-IV, and apo A-V), B (apo B48 and apo B100), C (apo C-I, apo C-II, apo C-III, and apo C-IV), D, E and H. The lipoprocessing proteins include lipoprotein lipase, hepatic triglyceride lipase, lecithin cholesteryl acyltransferase (LCAT) and cholesteryl ester transfer protein. The lipoprotein receptors include: LDL receptor, chylomicron remnant receptor and scavenger receptor. Mutations in the genes encoding these proteins may cause disturbances in lipoprotein metabolism that may lead to disorders including premature atherosclerosis. A particular disease may result from rare single-gene mutations (major gene effects) while another may be due to an accumulation of common mutations in several different genes each having small effect (some with no effect) and unable to cause disease on their own (polymorphisms).

Apo E polymorphisms appear to be importantly associated with variations in lipid and lipoprotein levels. Apo E has three different protein forms: E2, E3 and E4 differing from each other by a single amino acid substitution. Each isoform is encoded by distinct alleles on human chromosome 19. The presence of the E4 isoform is associated with coronary heart disease (Song et al. Ann of Int Med. 2004; 141(2):137-147). E2 is associated with the genetic disorder type III hyperlipoproteinemia and with both increased and decreased risk for atherosclerosis.

Other genetic polymorphisms have also been associated with atherosclerosis. Studies have suggested an association of common polymorphisms in the hepatic lipase gene, including LIPC-480C/T and LIPC-514C/T, with lipid levels and/or risk of CAD 5-lipoxygenase polymorphisms are thought to promote atherosclerosis by increasing leukotriene production within plaques. Genes that regulate the renin angiotensin system may also play a role in developing cardiovascular system disorders. The presence of the "deletion" (D) allele in the angiotensin converting enzyme (ACE) gene is associated with coronary artery disease (Tanriverdi et al. Hea Ves 2007;22(1):1-8).

The polymorphisms can be detected using any suitable commercially available kit or known method in the art including, but not limited to, allele-specific PCR, hybridization with an oligonucleotide probe, DNA sequencing, or enzymatic cleavage.

*Biomarkers.* A biomarker analysis is determined from information gathered relating to levels of circulating serum biomarkers, including, but not limited to, CRP, Lp-PLA2, N-terminal BNP and urinary thromboxane A2. Clinical measurements of biomarkers in serum may be performed by any acceptable method, including ELISA (See generally: Wang et al. Expert Rev. Mol. Diagn 2007;7(6):793-804; Dotsenko et al. Expert Rev Mol Diagn 2007;7(6):693-697).

One assay to measure CRP in CVD risk assessment (highly sensitive CRP or "hsCRP") is well known (Pearson et al. Circ 2003:107:499-511). HsCRP results should only be used in the absence of overt inflammatory processes, where results greater than 10 mg/L suggest the presence of an acute inflammatory process. Two measurements should be made at least 2 weeks apart. The findings to be interpreted are as follows: Low Risk <1.0 mg/L; Average Risk 1.0 to 3.0 mg/L; High Risk >3.0 mg/L.

Lp-PLA2 can be measured using ELISA (e.g., diaDexus PLAC Test). The assay system utilizes monoclonal anti-Lp-PLA2 antibodies (2C10) directed against Lp-PLA2 for solid phase immobilization on the microwell strips. Sample is added to the plate and incubated for 10 minutes at 20-26 °C. A second monoclonal anti-Lp-PLA2 antibody (4B4) labeled with the enzyme horseradish peroxidase (HRP) is then added and reacted with the immobilized antigen at 20-26 °C for 180 minutes, resulting in the Lp-PLA2 molecules being captured between the solid phase and the enzyme-labeled antibodies. The wells are washed with a supplied buffer to remove any unbound antigen. The substrate, tetramethylbenzidine (TMB), is then added and incubated at 20-26 °C for 20 minutes, resulting in the development of a blue color. Color development is stopped with the addition of Stop Solution, changing the color to yellow. The absorbance of the enzymatic turnover of the substrate is determined using a spectrophotometer at 450 nm and is directly proportional to the concentration of Lp-PLA2 present. A set of Lp-PLA2 calibrators is used to plot a standard curve of absorbance versus Lp-PLA2 concentration from which the Lp-PLA2 concentration in the test sample can be determined. The expected values are measured in ng/mL. Average value for females is 174 ng/mL (range 5th-95th percentile: 120-342), and the average value for males is 251 (range 5th-95th percentile: 131-376).

In one aspect, the biomarkers analyzed include one or more biomarker selected from HSCRP, Lp-PLA-2, and N-terminal proBNP.

*Atherosclerosis Imaging.* Information on atherosclerotic risk can also be collected using imaging tools. These tools may include conventional angiography, computed tomographic angiography, duplex ultrasonography (US) and magnetic resonance (MR) angiography.

A pharmaceutical composition for use in a method for treating diabetes in a subject in need thereof is disclosed. In some examples, there is disclosed a pharmaceutical composition for use in a method for treating a subject newly diagnosed with diabetes. In some examples, there is disclosed a composition for use in a method for delaying the onset of diabetes in a subject at risk thereof. In some embodiments, the compositions for use in methods comprise administering to the subject a food composition, a pharmaceutical composition, or a dietary supplement comprising 23-OH UA. A subject may be diagnosed with or is at risk of developing diabetes, such as type I, type II, or gestational diabetes. Type 1 diabetes signs and symptoms typically include increased thirst and frequent urination, extreme hunger, weight loss, fatigue and/or blurred vision. Type I diabetes testing and diagnosis may be based on at least one of the following: (a) Glycated hemoglobin (A1C) test, where the average blood sugar levels for the two-three months prior to the test is assessed based on the measurement of the percentage of blood sugar attached to hemoglobin. An A1C level of 6.5 percent or higher on two separate tests is usually indicative of diabetes. A result between 5.7 and 6.4 percent is considered pre-diabetes, and indicates a high risk of developing diabetes; (b) Random blood sugar test. Blood sugar values are typically expressed in milligrams per deciliter (mg/dL) or millimoles per liter (mmol/L). Regardless of the type of food consumed by the tested individual prior to the test, a random blood sugar level of 200 mg/dL (11.1 mmol/L) or higher is suggestive of diabetes, especially when coupled with any of the signs and symptoms of diabetes as noted above. A level between 140 mg/dL (7.8 mmol/L) and 199 mg/dL (11.0 mmol/L) is considered pre-diabetes, and indicates a high risk of developing diabetes; or (c) Fasting blood sugar test. A blood sample is taken after an overnight fast. A fasting blood sugar level less than 100 mg/dL (5.6 mmol/L) is considered normal. A fasting blood sugar level from 100 to 125 mg/dL (5.6 to 6.9 mmol/L) is considered pre-diabetes. A fasting blood sugar level of 126 mg/dL (7 mmol/L) or higher on two separate tests is usually indicative of diabetes.

In some embodiments, pharmaceutically acceptable salts of 23-OH UA are used. Non-limiting examples of suitable salts include sodium and potassium salts. Pharmaceutically acceptable salts utilized according to embodiments of the present invention are salts that do not substantially contribute to the toxicity of the compound. Such salts can be formed by well-known procedures.

23-OH UA may be administered in combination with another therapeutic or dietary agent, for example, an anti-diabetic agent. As used herein, "in combination" includes both sequential and concurrent administration of the different active agents. In certain embodiments 23-OH UA is co-formulated with a therapeutic or dietary agent. The pharmaceutical composition for use in the method of the present invention may be combined with additional treatment or treatments.

Pharmaceutical or supplement compositions of the present invention are preparations of one or more active ingredients with other chemical or natural components such as physiologically acceptable carriers and excipients, or plant/herb compositions or extracts. The purpose of a composition is to facilitate administration of a compound to an organism.

As used herein, the phrases "physiologically acceptable carrier" and "pharmaceutically acceptable carrier", which may be used interchangeably, refer to a carrier or a diluent that does not cause significant irritation to an organism and does not abrogate the biological activity and properties of the administered active agent.

As used herein, the term "excipient" refers to an inert substance added to a pharmaceutical composition to further facilitate administration of an active ingredient. Non-limiting examples of excipients include calcium carbonate, calcium phosphate, various sugars and types of starch, cellulose derivatives, gelatin, vegetable oils, and polyethylene glycols. Techniques for formulation and administration of drugs may be found in "Remington's Pharmaceutical Sciences", Mack Publishing Co., Easton, Pa., (Remington: The Science and Practice of Pharmacy, Gennaro, A., Lippincott, Williams & Wilkins, Philadelphia, Pa., 20th ed, 2000). Pharmaceutical compositions of the present invention may be manufactured by processes well known in the art, e.g., by means of conventional mixing, dissolving, granulating, dragee-making, levigating, emulsifying, encapsulating, entrapping, or lyophilizing processes. Pharmaceutical compositions for use in accordance with the present invention thus may be formulated in conventional manner using one or more physiologically acceptable carriers comprising excipients and auxiliaries, which facilitate processing of the active ingredients into preparations that can be used pharmaceutically. Proper formulation is dependent upon the route of administration chosen.

The pharmaceutical compositions of the invention are particularly suitable for administration systemically. Systemic administration includes all enteral and parenteral routes. Non-limiting examples of suitable administration routes include oral, rectal, transmucosal such as transnasal and buccal, intravenous, intramuscular, transdermal, subcutaneous, intradermal, intravesicular and inhalation routes. Typically, the pharmaceutical compositions of the present invention are administered by an oral route of administration.

For oral administration, the pharmaceutical composition can be formulated readily by combining the active compounds with acceptable carriers well known in the art. Such carriers enable the pharmaceutical composition to be formulated as tablets, pills, dragees, capsules, liquids, gels, syrups, slurries, suspensions, and the like, for oral ingestion by a patient. Preparations for oral use can be made using a solid excipient, optionally grinding the resulting mixture, and processing the mixture of granules, after adding suitable auxiliaries as desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, and sodium carbomethylcellulose; and/or physiologically acceptable polymers such as polyvinylpyrrolidone (PVP). If desired, disintegrating agents, such as cross-linked polyvinyl pyrrolidone, agar, or alginic acid or a salt thereof, such as sodium alginate, may be added. Pharmaceutical compositions that can be used orally include push-fit capsules made of gelatin, as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate, and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration. For buccal administration, the pharmaceutical compositions may take the form of tablets or lozenges formulated in conventional manner.

Pharmaceutical or supplement compositions suitable for use in the context of the present invention include compositions wherein the active ingredients are contained in an amount effective to achieve the intended purpose. Determination of an effective amount is well within the capability of those skilled in the art, especially in light of the detailed disclosure provided herein. Various supplements are readily available as nonprescription dietary supplements and are often recommended in natural health food stores or books for self-treatment of symptoms related to a disease or condition.

In certain aspects, the active ingredients of the pharmaceutical composition include approximately 1, 10, 100, to 500 mg, including all ranges and values there between, of 23-OH UA or a pharmaceutically acceptable salt thereof. The active ingredients of the pharmaceutical composition may include approximately 0.1, 1.0, 10, 20, 40, 60, to 80% by weight of 23-OH UA.

The compounds described herein can be administered in combination with other therapies or treatments.

In still other embodiments 23-OH UA can be used as an animal feed supplement. 23-OH UA can be admixed with animal feed. Accordingly, the present invention provides an animal feed supplement. In certain aspects the 23-OH UA accounts for at least 0.1, 1, and up to 10% by weight of the total feed composition; up to 30 % by weight is disclosed herein. The feed supplement of the present invention may be incorporated into the food of the animal and according to another aspect of the present invention there is provided an animal feed comprising a feed supplement as herein before defined.

### II. Examples

The following examples as well as the figures are included to demonstrate preferred embodiments of the invention. It should be appreciated by those of skill in the art that the techniques disclosed in the examples or figures represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

### METHODS

***Cell Culture*/*Priming.*** Human monocytic THP-1 cells (ATCC) were cultured in THP-1 medium (RPMI 1640, 5 mM glucose, 10% fetal bovine serum (FBS), 2% glutamax, 1% penicillin/streptomycin, 1% HEPES, 0.1% β-mercaptoethanol). Cells are spun down at 400g, 5 minutes at 25°C and resuspended in media at 0.5 x 106 cells/mL. Following 48 hours at 37°C and 5% CO2, cells are plated in 12-well cell culture plates. Phytochemicals are solubilized in dimethylsulfoxide (DMSO) and are added to suspended THP-1 cells at concentrations from 0.03 µM to 3 µM. Control cells without phytonutrients are treated with 0.1% DMSO. Cytotoxicity was assessed with a trypan exclusion assay. To induce a metabolically stressed condition (priming), cells were treated with freshly isolated human LDL is added (100 µg/ml) along with D-glucose (20 mM) for 24 hours.

***Chemotaxis.*** Chemotaxis assays are performed using 48-well, modified Boyden chambers (NeuroProbe, Gaithersburg, MD) as described previously (Ullevig et al. Redox Biology. 2014 01/03;2:259-66). Briefly, monocytes are primed with glucose and LDL in the presence of UA, 23-OHUA or vehicle (0.1% DMSO). Cells are loaded into upper wells of the chemotaxis chamber. Lower wells contain cell media with 1 nM MCP-1 (R&D Systems, Minneapolic, MN) or vehicle. A 5 µm polyvinyl pyrrolidone-free polycarbonate filter membrane islayered between the upper and lower chambers, and the chamber is incubated for 1.5 h for THP-1 monocytes at 37 °C and 5% CO2. The membrane is washed and cells removed from the upper side of the filter and fixed with methanol. Transmigrated are stained with 1 µM propidium iodide. Fluorescence intensity, which correlates with cell number is quantified with KODAK Image Station 4000MM (Carestream, Rochester, NY).

***MKP-1 Activity Assay.*** MKP-1 activity assay was performed as described previously (Kim et al., PNAS. 2012 October 09;109(41):E2803-12). Briefly cell lysates were analyzed in the presence or absence of 40 µM sanguinarine, a specific inhibitor of MKP-1. Sanguinarine-sensitive PTP activity was attributed to MKP-1. Assays were initiated by adding 100 µM of phosphotyrosine peptide substrate to cell extracts (2 µg protein) diluted in 20 mM Tris-HCl (pH 7.5), 150 mM NaCl, 1% Nonidet P-40 and warmed to 30 °C. The reaction was stopped after 10 min. MKP-1 activity was assayed spectrophotometrically as the amount of inorganic phosphate released using a VersaMax reader (Molecular Devices). Phosphate released by MKP-1 was quantified from a standard curve prepared with known amounts of KH₂PO₄.

***Animals.*** Female LDL-R^{-/-} recipient mice (B6.129S7-Ldlr^{tm1her}/J, stock no. 002207) were obtained from Jackson Labs (Bar Harbor, ME). All mice were maintained in colony cages on a 12-h light/12-h dark cycle. Mice were randomly assigned to one of four groups, (8 mice per group at 6 and 20 weeks) high fat diet (HFD; 21% milk fat and 0.2% cholesterol, diet no. F5540, Bio-Serv, Frenchtown, NJ), HFD supplemented with 0.05% ursolic acid, HFD supplemented with 0.05% 23-OHUA or maintenance diet (MD; AIN-93G, Bio-Serv). Phytonutrient diets were prepared by Bio-Serv. Mice were maintained on diets for 6 or 20 weeks. Fasted body weights were assessed weekly and fasted glucose was assessed by venous tail bleed biweekly. At 6 and 20 weeks, complete blood counts were run using whole blood. All studies were performed in accordance with the guidelines and regulations of and with the approval of the UTHSCSA Institutional Animal Care and Use Committee.

***Athero (en face).*** After peritoneal lavage, the chest cavity was opened and the heart and aorta were perfused via the left ventricle with 10 ml PBS followed by 10 ml of ice-cold 4% paraformaldehyde (PFA) in PBS. With the heart intact, the entire aorta (extending 5 mm after bifurcation of the iliac artery, including the subclavian artery, right, and left carotid arteries) was dissected free of fat and removed. Hearts were separated from the aorta and embedded in Tissue-Tek^{®} Optimal Cutting Temperature compound (OCT; SAKURA Finetek USA, Inc., Torrance, CA) in a plastic cryosection mold.

The aortas (proximal ascending aorta to the bifurcation) were fixed in 4% PFA prior to staining for *en face* analysis. To determine the extent of the atherosclerosis, aortas were stained with Oil Red O (ORO; Sigma-Aldrich, St. Louis, MO) and digitally imaged with a camera connected to a dissecting microscope (Leica). Images were taken to include the entire aorta. Next, the aortas were opened longitudinally and digitally imaged at fixed magnification. Total aortic area and lesion area were calculated using Image Pro Plus (version 6.3; Media Cybernetics, Warrendale, PA) and ImageJ (version 1.47; NIH, Bethesda, Maryland). Lesion area is expressed as a percent of total aortic area ± S.E.

***Monocyte Subsets**.* For the identification and quantification of monocyte subsets, whole blood was incubated in FACS buffer at 4°C, for 15 min to block F_{C} receptors (CD16/CD32). Red blood cells (RBC) were lysed with 2 ml of BD FACS lysing solution (BD Biosciences) and subsequently labeled with V450-ly-6G (BD Horizon), APC-efluor 780 ly-6C (eBioscience), PE-CD115 (eBioscience), and Alexa Fluor 488-CD11b (BioLegend) antibodies in FACS buffer. Cells were fixed and permeabilized with 2% PFA in PBS for 30 min at 4°C. Analysis was performed using a BD LSR-II.

***Matrigel Plug Assay.*** The matrigel plug assay was used to determine monocyte chemotaxis in response to chemoattractant (MCP-1) *in vivo.* Matrigel (BD Biosciences) supplemented with vehicle (injected in mouse's left flank) or MCP-1 (300 nM, injected in mouse's right flank) is injected subcutaneously. After 3 days, mice were sacrificed and the plugs removed. Plugs are dissolved in collagenase for 2 hours at 37°C. Cells were stained with calcein/AM (Invitrogen, Grand Island, NY) and counted using an automated fluorescent cell counter (Nexcelcom Bios, Lawrence, MA).

***Statistics.*** Data were analyzed using ANOVA (Sigma Stat 12.0). Data were tested for use of parametric or nonparametric post hoc analysis, and multiple comparisons were performed by using the Least Significant Difference method. All data are presented as mean ± SE. Results were considered statistically significant at the P<0.05 level.

All of the methods disclosed and claimed herein can be made and executed without undue experimentation in light of the present disclosure.

The scope of the invention is defined by the appended claims.

## Claims

1. A pharmaceutical composition comprising 23 hydroxy ursolic acid or a pharmaceutically acceptable salt thereof for use in a method of reducing the risk of vascular disease by attenuating monocyte hypersensitivity in a subject, the use comprising:
administering to the subject an effective amount of the pharmaceutical composition comprising the 23 hydroxy ursolic acid or the pharmaceutically acceptable salt thereof, wherein monocyte hypersensitivity is attenuated.

2. The pharmaceutical composition for use of claim 1, wherein the vascular disease is selected from atherosclerosis, peripheral artery disease, coronary heart disease (CHD), or cardiovascular disease (CVD).

3. The pharmaceutical composition for use of claim 1, wherein the subject is administered about 1 to 500 mg of 23 hydroxy ursolic acid or salt thereof per kilogram of body weight.

4. The pharmaceutical composition for use of claim 1, wherein the method further comprises administering an anti-atherogenic therapy in combination with the 23 hydroxy ursolic acid.

5. The pharmaceutical composition for use of claim 1, wherein the subject is human.

6. The pharmaceutical composition for use of claim 5, wherein the subject is younger than 18 years of age.

7. The pharmaceutical composition for use of claim 1, wherein the subject is an animal; wherein, optionally, the animal is a pet or livestock.

8. A dietary supplement comprising 23 hydroxy ursolic acid or a salt thereof for use in a method of reducing the risk of vascular disease by attenuating monocyte hypersensitivity in a subject, the use comprising: administering to the subject an effective amount of the dietary supplement comprising the 23 hydroxy ursolic acid or the salt thereof, wherein monocyte hypersensitivity is attenuated.

9. A dietary supplement comprising 10 to 90% by weight 23 hydroxy ursolic acid.

10. The dietary supplement of claim 9 for use in a method of supplementing a diet of an animal.

11. An animal food composition comprising 0.1 to 10% by weight of 23 hydroxy ursolic acid.

12. The composition of claim 11, wherein the composition is a pet or livestock food.

## Patentansprüche

1. Pharmazeutische Zusammensetzung, die 23-Hydroxyursolsäure oder ein pharmazeutisch verträgliches Salz davon umfasst, für die Verwendung in einem Verfahren zur Verringerung des Risikos von Gefäßerkrankung durch Abschwächung von Hypersensitivität von Monozyten bei einem Subjekt, wobei die Verwendung Folgendes umfasst:
Verabreichen an das Subjekt einer wirksamen Menge der pharmazeutischen Zusammensetzung, die 23-Hydroxyursolsäure oder das pharmazeutisch verträgliche Salz davon umfasst, wobei Hypersensitivität von Monozyten abgeschwächt wird.

2. Pharmazeutische Zusammensetzung für die Verwendung nach Anspruch 1, wobei die Gefäßerkrankung aus Atherosklerose, peripherer arterieller Erkrankung, koronarer Herzkrankheit (CHD) oder kardiovaskulärer Erkrankung (CVD) ausgewählt ist.

3. Pharmazeutische Zusammensetzung für die Verwendung nach Anspruch 1, wobei dem Subjekt etwa 1 bis 500 mg 23-Hydroxyursolsäure oder eines Salzes davon pro Kilogramm Körpergewicht verabreicht wird.

4. Pharmazeutische Zusammensetzung für die Verwendung nach Anspruch 1, wobei das Verfahren weiter das Verabreichen einer antiatherogenen Therapie zusammen mit der 23-Hydroxyursolsäure umfasst.

5. Pharmazeutische Zusammensetzung für die Verwendung nach Anspruch 1, wobei das Subjekt ein Mensch ist.

6. Pharmazeutische Zusammensetzung für die Verwendung nach Anspruch 5, wobei das Subjekt jünger als 18 Jahre ist.

7. Pharmazeutische Zusammensetzung für die Verwendung nach Anspruch 1, wobei das Subjekt ein Tier ist;
wobei optional das Tier ein Haustier oder Nutztier ist.

8. Nahrungsergänzungsmittel, das 23-Hydroxyursolsäure oder ein Salz davon umfasst, für die Verwendung in einem Verfahren zur Verringerung des Risikos von Gefäßerkrankung durch Abschwächung von Hypersensitivität von Monozyten bei einem Subjekt, wobei die Verwendung Folgendes umfasst: Verabreichen an das Subjekt einer wirksamen Menge des Nahrungsergänzungsmittels, das 23-Hydroxyursolsäure oder das Salz davon umfasst, wobei Hypersensitivität von Monozyten abgeschwächt wird.

9. Nahrungsergänzungsmittel, das 10 bis 90 Gewichts-% 23-Hydroxyursolsäure umfasst.

10. Nahrungsergänzungsmittel nach Anspruch 9 für die Verwendung in einem Verfahren zur Ergänzung einer Nahrung eines Tiers.

11. Tierfutterzusammensetzung, die 0,1 bis 10 Gewichts-% 23-Hydroxyursolsäure umfasst.

12. Zusammensetzung nach Anspruch 11, wobei die Zusammensetzung ein Futter für ein Haustier oder Nutztier ist.

## Revendications

1. Composition pharmaceutique comprenant de l'acide 23-hydroxy-ursolique ou un sel pharmaceutiquement acceptable de celui-ci, destinée à être utilisée dans un procédé de réduction du risque de maladie vasculaire en atténuant l'hypersensibilité des monocytes chez un sujet, l'utilisation comprenant :
l'administration au sujet d'une quantité efficace de la composition pharmaceutique comprenant l'acide 23-hydroxy-ursolique ou le sel pharmaceutiquement acceptable de celui-ci, dans laquelle l'hypersensibilité des monocytes est atténuée.

2. Composition pharmaceutique destinée à être utilisée selon la revendication 1, dans laquelle la maladie vasculaire est choisie parmi une athérosclérose, une maladie artérielle périphérique, une maladie coronarienne (CHD), ou une maladie cardiovasculaire.

3. Composition pharmaceutique destinée à être utilisée selon la revendication 1, dans laquelle on administre au sujet d'environ 1 à 500 mg d'acide 23-hydroxy-ursolique ou sel de celui-ci par kilogramme de poids corporel.

4. Composition pharmaceutique destinée à être utilisée selon la revendication 1, dans laquelle le procédé comprend en outre l'administration d'un traitement antiathérogène en combinaison avec l'acide 23-hydroxy-ursolique.

5. Composition pharmaceutique destinée à être utilisée selon la revendication 1, dans laquelle le sujet est un humain.

6. Composition pharmaceutique destinée à être utilisée selon la revendication 5, dans laquelle le sujet a un âge inférieur à 18 ans.

7. Composition pharmaceutique destinée à être utilisée selon la revendication 1, dans laquelle le sujet est un animal ; dans laquelle, éventuellement, l'animal est un animal de compagnie ou du bétail.

8. Complément alimentaire comprenant l'acide 23-hydroxy-ursolique ou un sel de celui-ci destiné à être utilisé dans un procédé de réduction du risque d'une maladie vasculaire en atténuant l'hypersensibilité des monocytes chez un sujet, l'utilisation comprenant : l'administration au sujet d'une quantité efficace du complément alimentaire comprenant l'acide 23-hydroxy-ursolique ou un sel de celui-ci, dans lequel l'hypersensibilité des monocytes est atténuée.

9. Complément alimentaire comprenant de 10 à 90 % en poids de l'acide 23-hydroxy-ursolique.

10. Complément alimentaire selon la revendication 9 destiné à être utilisé dans un procédé pour compléter le régime alimentaire d'un animal.

11. Composition alimentaire pour animaux comprenant de 0,1 à 10 % en poids de l'acide 23-hydroxy-ursolique.

12. Composition selon la revendication 11, dans laquelle la composition est un aliment pour animaux de compagnie ou pour le bétail.
